**Europäisches Patentamt**

**European Patent Office**

**Office européen des brevets**

(19)

(11) Veröffentlichungsnummer: **0 049 469**
**B2**

(12) **NEUE EUROPÄISCHE PATENTSCHRIFT**

(45) Veröffentlichungstag der neuen Patentschrift:
26.10.88

(51) Int. Cl.⁴: **A 61 L 15/04**

(21) Anmeldenummer: **81107775.9**

(22) Anmeldetag: **30.09.81**

(54) **Kollagene Wundauflage.**

(30) Priorität: **03.10.80 DE 3037513**

(43) Veröffentlichungstag der Anmeldung:
**14.04.82 Patentblatt 82/15**

(45) Bekanntmachung des Hinweises auf die Patenterteilung:
**23.11.83 Patentblatt 83/47**

(45) Bekanntmachung des Hinweises auf die Entscheidung
über den Einspruch:
**26.10.88 Patentblatt 88/43**

(84) Benannte Vertragsstaaten:
**AT BE CH DE FR GB IT LI LU NL SE**

(56) Entgegenhaltungen:
DE - A - 1 570 169
DE - A - 2 625 289
DE - A - 2 823 620
DE - A - 2 843 963
US - A - 4 089 333

**Med. Welt, Bd.29, Heft 17, (1978), S. 720-724
Thrombos.Hemostas. 38 (1977), S.191
Blut 39 (1979), S.64, Vortrag 1.5.2
"Fibrinogen, Fibrin und
Fibrinkleber",Verhandlungsbericht der Deutschen
Arbeitsgemeinschaft für Blutgerinnungsforschung über
die 23. Tagung in Heidelberg, Febr.1979 (Verlag F.K.
Schattauer, N.Y.), S.257, 271-272, 291**

(73) Patentinhaber: **Dr. Ruhland Nachf. GmbH, Stadtplatz 7,
D-8425 Neustadt/Donau (DE)**

(72) Erfinder: **Stemberger, Axel, Dr. rer. nat.,
Cramer-Klett-Strasse 35e, D-8014 Neubiberg (DE)**

(74) Vertreter: **Hansen, Bernd, Dr.rer.nat. et al, Hoffmann,
Eitle & Partner Patentanwälte Arabellastrasse 4,
D-8000 München 81 (DE)**

(56) Entgegenhaltungen: (Fortsetzung)
**"Collagen-Platelet Interaction", Proceedings of the
First Munich Symposium on Biology of Connective
Tissue (Juli 1976), S. 379-382 u. 397-398
Wiener Med.Wochenzeitschr. Nr. 7/1976, S. 86-89**

## Beschreibung

Kollagen wird seit geraumer Zeit in der Chirurgie verwendet. Es kann in Form von Schwämmen oder Fasern zur Blutstillung verwendet werden und ist auch nach entsprechender Modifizierung zur Beschleunigung der Wundheilung geeignet. Bei Patienten mit defekter Blutgerinnung oder bei grossflächigen Blutungen genügen übliche kollagene Wundauflagen nicht. Man hat daher bereits versucht, Kollagenmaterial mit dem Gewebe zu verkleben, wobei man Kleber auf Basis Resorcin-Formaldehyd, Kollagen oder Gelatine verwendete. Solche Kleber sind zwar hämostyptisch, jedoch wegen ihrer Toxizität für die Praxis ungeeignet. Dies gilt auch für Acrylatkleber und die Kombination mit kollagenen Wundauflagen.

Es ist bekannt, dass Kollagen kovalente Vernetzungen mit Bindegewebebestandteilen eingehen kann. Dabei finden Vernetzungen vom Kollagen über Schiff-Basen sowie Aldolkondensation statt. Bekannt ist auch, dass bei Basalmembranen die Gewebefestigkeit zusätzlich durch S-S-Brücken des Basalmembrankollagens erreicht wird. Bekannt ist weiterhin, Proteine, wie Albumin mit intermolekularen S-S-Bindungen nach milder Reduktion und nachfolgender Oxidation, über S-S-Brücken zu vernetzen.

Bei Verletzungen bildet sich durch die Blutgerinnung ein primärer Wundverschluss. Hierfür sind aggregierte Thrombozyten und ein Fibrinnetzwerk verantwortlich. Es ist bekannt, dass einzelne Fibrinmoleküle durch Transglutaminase vernetzt werden. Hierbei bilden sich neue Peptidbindungen zwischen Glutaminsäure und Lysin zwischen benachbarten Ketten. Mit der Technik der Fibrinklebung, also durch Verwendung von Fibrinogen und Thrombin, wird die Endphase der plasmatischen Blutgerinnung nachgeahmt.

Die Fibrinklebung allein ist nicht in der Lage, grossflächige Blutungen zu stillen. Dies gelingt erst durch eine kombinierte Anwendung der Fibrinklebung mit einer resorbierbaren kollagenen Wundauflage. Jedoch muss man dabei drei Komponenten bereithalten, nämlich die kollagene Wundauflage, Thrombinlösung mit Antifibrinolytika und eine tiefgefrorene hochkonzentrierte Fibrinogenlösung. Da Blutungen häufig plötzlich und unprogrammiert auftreten, stehen diese drei Komponenten im entscheidenden Augenblick häufig nicht anwendungsbereit zur Verfügung, denn zumindest die tiefgefrorene Fibrinogenlösung muss zuvor aufgetaut werden. Auch das Mischen vor der Applikation ist verhältnismässig kompliziert.

Aufgabe der Erfindung ist es, eine kollagene Wundauflage zur Verfügung zu stellen, die mit dem Gewebe verklebt und die bekannten Nachteile der Fibrinklebung in Kombination mit resorbierbarem Kollagen nicht aufweist. Verbunden mit dieser Aufgabe ist eine Verbesserung der kollagenen Wundauflagen zur lokalen Hämostase.

Zur Lösung dieser Aufgabe dient eine gewebeverklebbare kollagene Wundauflage, die dadurch gekennzeichnet ist, dass Kollagen und Fibrinogen durch Gefriertrocknung miteinander kombiniert worden sind.

Die erfindungsgemässen kollagenen Wundauflagen können in an sich bekannter Weise mit Arzneimittelwirkstoffen beladen werden.

Das als Wundauflage verwendete Kollagen liegt in der üblichen Form von Wundauflagen, also als Netz, Gewebe, Schwamm und dgl. vor. Solche Wundauflagen sind aus dem Stand der Technik bekannt.

Das verwendete Kollagen zeigt eine Reinheit, ausgedrückt durch den Faktor Stickstoff:Hydroxyprolin < 4 vorzugsweise < 3. Da Hydroxyprolin nur in Kollagen vorkommt, ist dies ein Mass für die Reinheit des Kollagens.

Das Fibrinogen ist in der gewebeverklebbaren kollagen Wundauflage in einer Menge von 0,5 bis 10 mg/cm² vorzugsweise 4 bis 6 mg/cm² enthalten.

Bekannt ist, dass Kollagen als Träger für Antibiotika, wie Gentamycin, geeignet ist. Auch Tetracyclin oder weitere Antibiotika oder Chemotherapeutika können in das modifizierte Kollagen eingearbeitet werden. Dies ist ein zusätzlicher Effekt, der bei den erfindungsgemässen Wundauflagen erzielt werden kann.

Herstellung von Kollagenen:

Von allen Pigmentschichten und Muskelresten befreite frische Rindersehnen wurden homogenisiert und eine Menge, die 100 g Trockengewicht entspricht, in 3 l 0,05 m Citratpuffer (pH 3,7) 24 Stunden lang extrahiert und anschliessend gegen 1% Essigsäure 12 Stunden lang dialysiert.

Das in 3 l 1% Essigsäure suspendierte Gewebe wird 48 Stunden bei 15°C unter ständigem Rühren mit Pepsin im Verhältnis Kollagen:Pepsin = 50:1 inkubiert.

Der Ansatz wird mit 1% Essigsäure auf 5 Liter verdünnt und durch Zentrifugation von den ungelösten Sehnenfragmenten befreit.

Die viskose Kollagenlösung wird gegen alkalisiertes Leitungswasser (pH 8,0) dialysiert und dann scharf zentrifugiert. Der Rückstand wird erneut in 5 Liter 1% Essigsäure gelöst und dialysiert. Dieser Vorgang wird wiederholt, bis der Faktor Stickstoff:Hydroxyprolin < 3 ist. Nach dem letzten Dialysieren wird mittels 0,05% Essigsäure eine 1,5% Kollagenlösung hergestellt, die für die nachfolgenden Versuche Verwendung findet.

Herstellung von Fibrinogenlösungen:

Im Handel befindliche sterile Fibrinogenbulkware wird in sterilem Aqua dest. gelöst, so dass eine Lösung von 50 mg Fibrinogen/ml Lösung vorliegt, die für die nachfolgenden Versuche verwendet wird.

Beispiel 1

Herstellung einer Kollagen-Fribinogen-haltigen Wundauflage:

Von einer strahlensterilisierten 1,5%igen Kollagenlösung werden 10 ml unter aseptischen Bedingungen in eine sterile Flasche mit Septum gegeben und im Kältebad (Trockeneis–Äthanol) unter leichtem Rühren tiefgefroren. Nachdem etwa $2/3$ dieser Lösung gefroren sind, werden 5 ml einer Kollagen-Fibrinogenlösung (Kollagen:Fibrinogen = 1:1) zugegeben und erneut tiefgefroren, bis $2/3$ dieser Lösung tiefgefroren sind. Dann werden 5 ml der Fibrinogenlösung zugegeben, tiefgefroren und lyophilisiert.

Beispiel 2
Herstellung gentamycinhaltiger Wundauflagen:

Zur Herstellung gentamycinhaltiger Wundauflagen werden 100 mg Gentamycin zu 100 ml einer 1%igen Kollagenlösung gegeben und diese Lösung wie vorher beschrieben zur Herstellung der kollagen-fibrinogenhaltigen Wundauflage verwendet.

Beispiel 3
Herstellung von Wundauflagen, die Kollagen und Fibrinogen enthalten:
Es wird zunächst 100 ml einer 0,5–1%igen Kollagenlösung in eine Metallform von 10×10 cm gegeben, nach üblicher Technik gefriergetrocknet und der resultierende Schwamm dann sterilisiert. Unter aseptischen Bedingungen wird nun dieser sterilisierte Kollagenschwamm mit einer Fibrinogenlösung besprüht, wobei pro Quadratzentimeter Kollagenoberfläche 0,5–10 mg Fibrinogen aufgetragen werden. Anschliessend erfolgt eine erneute Gefriertrocknung und Verpackung unter sterilen Kautelen.
Bei Anwendung der erfindungsgemässen modifizierten gewebeverklebbaren kollagenen Wundauflagen in der Praxis findet die Resorption innerhalb klinisch angemessener Zeiträume statt. Erfindungsgemäss können grossflächige Wunden insbesondere im Bauchbereich versorgt werden.

## Patentansprüche

1. Gewebeverklebbare kollagene Wundauflage aus einer Kombination von Kollagen mit Fibrinogen, die Arzneimittelwirkstoffe enthalten kann, dadurch gekennzeichnet, dass Kollagen und Fibrinogen durch Gefriertrocknung kombiniert worden sind.
2. Gewebeverklebbare kollagene Wundauflage gemäss Anspruch 1 dadurch gekennzeichnet, dass der Arzneimittelwirkstoff ein Antibiotikum ist.
3. Gewebeverklebbare kollagene Wundauflage gemäss Anspruch 2, dadurch gekennzeichnet, dass das Antibiotikum Gentamyzin ist.

4. Gewebeverklebbare kollagene Wundauflage, gemäss Anspruch 1, dadurch gekennzeichnet, dass das Kollagen in Form eines Gewebes oder eines Schwammes vorliegt.
5. Gewebeverklebbare kollagene Wundauflage gemäss Anspruch 1, dadurch gekennzeichnet, dass das Kollagen eine Reinheit ausgedrückt durch den Faktor Stickstoff:Hydroxyprolin <4, vorzugsweise <3 hat.

## Claims

1. A tissue bonding collagenic surgical dressing consisting of a combination of collagen with fibrinogen, which can contain pharmaceutical active ingredients, characterized in that collagen and fibrinogen were combined by freeze drying.
2. A tissue bonding collagenic surgical dressing as set forth in claim 1, characterized in that the pharmaceutical active ingredient is an antibiotic.
3. A tissue bonding collagenic surgical dressing as set forth in claim 2, characterized in that the antibiotic is gentamycin.
4. A tissue bonding collagenic surgical dressing as set forth in claim 1, characterized in that the collagen is present in the form of a fabric or of a sponge.
5. Tissue bonding collagenic surgical dressing as set forth in claim 1, characterized in that the collagen has a purity expressed by the factor nitrogen:hydroxyprolin <4, preferably <3.

## Revendications

1. Pansement à base de collagène pouvant adhérer aux tissus, constitué par une combinaison de collagène et de fibrinogène, qui peut contenir une substance active thérapeutiquement, caractérisé en ce que le collagène et le fribrinogène sont combinés par lyophilisation.
2. Pansement à base de collagène pouvant adhérer aux tissus selon la revendication 1, caractérisé en ce que la substance active thérapeutiquement est un antibiotique.
3. Pansement à base de collagène pouvant adhérer aux tissus, selon la revendication 2, caractérisé en ce que l'antibiotique est la gentamycine.
4. Pansement à base de collagène pouvant adhérer aux tissus, selon la revendication 1, caractérisé en ce que le collagène se présente sous la forme d'un tissu ou d'une éponge.
5. Pansement à base de collagène pouvant adhérer aux tissus selon la revendication 1, caractérisé en ce que le collagène a un degré de pureté exprimé par le rapport azote:hydroxyproline inférieur à 4, de préférence inférieur à 3.